# EUROPÄISCHE PATENTANMELDUNG

(11) **EP 0 951 919 A2**
(43) Veröffentlichungstag der Anmeldung: **27.10.1999**
(21) Anmeldenummer: 99106648.1
(22) Anmeldetag: 31.03.1999
(51) Int. Cl.: A61M 25/01

(54) **Antriebsvorrichtung für eine elastische Sonde, insbesondere medizinische Sonde**

(30) Priorität: 03.04.1998 DE 19815119
(71) Anmelder: B. BRAUN MELSUNGEN AG, 34212 Melsungen (DE)
(72) Erfinder: Frimberger, Eckart Dr., 80804 München (DE)
(74) Vertreter: Körber, Wolfhart, Dr.

(57) **Zusammenfassung**

Eine Antriebsvorrichtung für eine elastische Sonde (5), insbesondere eine medizinische Sonde, weist eine Auflagefläche (6) für die Sonde (5), ein Rad (2) mit einer zirkulären konischen, sich zur Radmitte verjüngenden Nut (3) auf, wobei das Rad (2) so gegenüber der Auflagefläche (6) der Sonde (5) bewegbar ist, daß die Sonde (5) mit den Seitenwänden der konischen Nut (3) des Rades (2) gegen die Auflagefläche (6) andrückbar ist.

Die elastische Sonde (5) wird so in reibschlüssiger Verbindung mit dem Rad (2) gehalten und kann durch Drehen des Rades (2) mit geringem Kraftaufwand definiert in beiden Richtungen beweget werden.

## Beschreibung

Die Erfindung betrifft eine Antriebsvorrichtung für eine elastische Sonde, insbesondere eine medizinische Sonde, mit einem Handgriff und einem drehbaren Betätigungselement. Eine solche Vorrichtung ist beispielsweise aus der DE 297 17 110 U bekannt.

Derartige elastische Sonden werden in verschiedenen Bereichen der Medizin zu therapeutischen und diagnostischen Zwecken eingesetzt. Beispielsweise werden derartige Sonden mit einem Endoskop in den Magen-Darin-Trakt, in die Gallenwege und den Gang der Bauchspeicheldrüse des Körpers eingeführt. Die Sonde kann, in unterschiedlicher Ausformung, verschiedenen Zwecken dienen. Häufig werden Sonden in Form von Führungsdrähten verwendet, über welche diagnostische und therapeutische Instrumente ins Zielgebiet vorgeschoben werden. Andere Sonden dienen der Abtragung von Polypen (Polypektomie mit Polypektomie-Schlinge). Eine versteifbare drahtförmige Sonde ist in der Europäischen Patentanmeldung EP 92 923 276 (0 613 386) beschrieben.

Das Einführen und Herausziehen von Führungsdrähten in das Endoskop oder in Hohlsonden erfolgt manuell durch den Arzt oder Assistenzpersonal. Wird über einen plazierten Führungsdraht beispielsweise ein Drainagerohr (Stent, Endoprothese) durch das Endoskop in den Gallengang vorgeschoben, wozu ein ausreichend langer Schiebeschlauch (Pusher) verwendet wird, sind dabei infolge der Reibung der einzelnen Komponenten teilweise erhebliche Schubkräfte manuell aufzubringen. Die aufzubringende Schubkraft wird teilweise durch die geringe Reibung möglicherweise feuchter Behandlungshandschuhe beeinträchtigt, zudem ist die auf diese Weise erzielbare Schubkraft limitiert.

Bei der Sonde kann es sich auch um eine Polypektomie-Schlinge handeln. Diese besteht aus einer an einem Zugseil befestigten Drahtschlinge, die in einer Hohlsonde verschieblich ist und mittels eines Griffes aus der Hohlsonde ausgefahren und wieder eingezogen werden kann. Der Griff besteht in der Regel aus einem Ring für den Daumen (Daumenring) und einem Doppelring (alternativ Griffrolle) für Zeige- und Mittelfinger, der auf einer mit dem Daumenring fest verbundenen Schubstange hin und herbewegt werden kann. Der Griff wird mit einer Hand betätigt. Der maximale Hub ist durch die Handgröße des Bedienungspersonals begrenzt (Spanne zwischen Daumen- und Doppelring), so daß dadurch auch die Größe der Schlinge limitiert ist, was nachteilig ist.

Der vorliegenden Erfindung liegt daher die Aufgabe zugrunde, eine Antriebsvorrichtung für den Vorschub von Instrumenten, beispielsweise Drainagerohren, über Führungsdrähte vorzuschlagen, die einen geringen Kraftaufwand erfordert, und außerdem eine Betätigungsvorrichtung (Griff) für aus Hohlsonden auszufahrende Instrumente, beispielsweise Polypektomie-Schlingen vorzuschlagen, die bei Einhandbetätigung einen möglichst großen Hub ermöglicht und eine schnelle und selbsttätige Befestigung der zu manipulierenden Sonde an dem Betätigungselement ermöglicht.

Gelöst wird die Aufgabe durch eine Antriebsvorrichtung mit einer Auflagefläche für die Sonde und einem Rad mit einer zirkulären konischen, sich zur Radmitte hin verjüngenden Nut, wobei das Rad so gegenüber der Auflagefläche der Sonde bewegbar ist, daß die Sonde mit den Seitenwänden der konischen Nut gegen die Auflagefläche andrückbar ist. Die Sonde wird so im Reibschluß in der konischen Nut des Rades gehalten, daß durch Drehung des Rades die Sonde mitgeführt wird. Die Sonde kann daher durch Drehung des Rades definiert in die gewünschte Richtung bewegt werden. Bei feststehender Sonde (Führungsdraht) kann die Antriebsvorrichtung relativ zum Führungsdraht bewegt werden und dadurch zum Vorschub von Instrumenten benutzt werden.

Je größer das Verhältnis des Außenradius des Rades zu dem Radius des Berührungspunktes der Sonde mit der konischen Nut ist, desto größer ist die Hebelwirkung der Antriebsvorrichtung.

Das Rad kann mittels in einem Schlitz gehaltener äußerer Achsenbolzen oder mittels einer mittleren Achse bewegbar an einem Gehäuse angebracht sein. Der Schlitz zur Führung der Achse des Rades kann dabei senkrecht oder mit einem kleinen Winkel relativ zur Senkrechten auf die Auflagefläche angeordnet sein.

Vorzugsweise weist das Gehäuse der Antriebsvorrichtung Durchführungen für die drahtförmige Sonde auf. Die Auflagefläche für die Sonde kann durch einen Steg gebildet sein, dessen Breite Kleiner ist als der Außendurchmesser der Sonde. Der Steg kann zur besseren Führung der Sonde eine gekrümmte Auflagefläche haben.

Um verschiedene Sondenstärken benutzen zu können, kann das Rad durch ein anderes Rad mit anderer Nutbreite austauschbar am Gehäuse befestigt sein.

Der Winkel der Seitenwände der konischen Nut gegenüber der Senkrechten auf die Drehachse des Rades kann je nach Material und Steifigkeit der Sonde geeignet gewählt werden. Vorzugsweise liegt dieser zwischen 1° und 6°, insbesondere zwischen 3° und 4°.

Zur einfacheren Betätigung und zur Vergrößerung der Reibung kann das Rad auf seiner Umfangsfläche gerändelt sein.

Die erfindungsgemäße Antriebsvorrichtung wird im folgenden anhand bevorzugter Ausführungsbeispiele unter Bezugnahme auf die Zeichnungen beschrieben, in der
- Figur 1: eine schematische Seitenansicht eines ersten Ausführungsbeispiels der erfindungsgemäßen Antriebsvorrichtung ist;
- Figur 2: eine Aufsicht auf die in Figur 1 gezeigte Antriebsvorrichtung ist;
- Figur 3: eine schematische Querschnittsansicht entlang der Linie III-III von Figur 2 ist;
- Figur 4: eine schematische Seitenansicht eines zweiten Ausführungsbeispieles der erfindungsgemäßen Antriebsvorrichtung ist;
- Figur 5: eine Aufsicht auf die Antriebsvorrichtung von Figur 4 ist; und
- Figur 6: eine schematische Querschnittsansicht entlang der Linie VI-VI in Figur 5 ist.

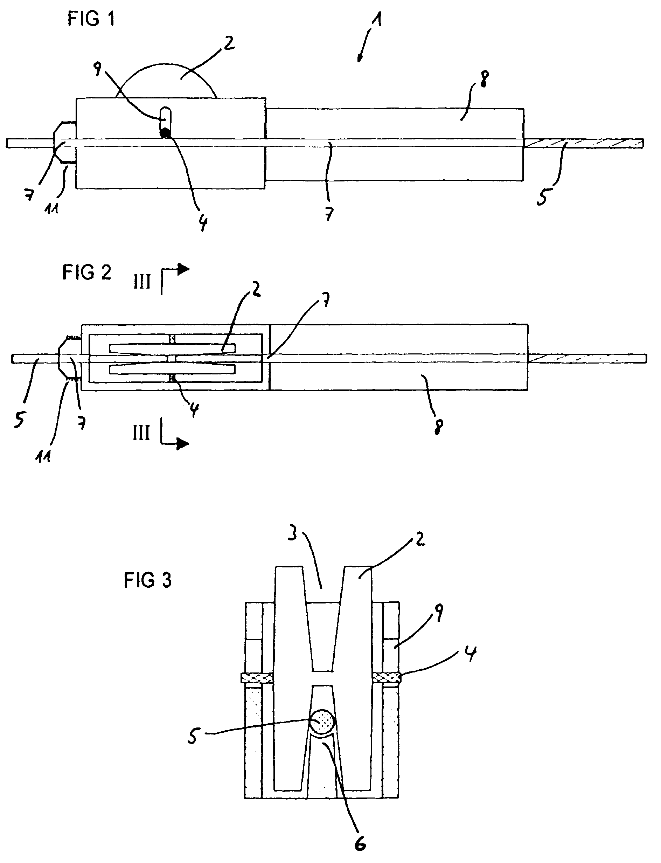

Figur 1 zeigt ein erstes Ausführungsbeispiel der erfindungsgemäßen Antriebsvorrichtung für eine elastische Sonde. Bei der Sonde kann es sich dabei um jegliche Art von langgestreckten medizinischen Sonden zum Einführen in den Körper handeln. Insbesondere können dies endoskopische Sonden in Form von Führungsdrähten oder Polypektomie-Schlingen sein. Die Sonde 5 kann dabei aus Kunststoff, Draht, als Hohlsonde mit gewickeltem Drahtmantel oder jedem anderen geeigneten Material ausgebildet sein. Die Sonde 5 wird durch eine vordere Öffnung 7 in die Antriebsvorrichtung, die insgesamt mit 1 bezeichnet ist, eingelegt und liegt auf einem Steg 6 auf. Wie in Figur 3 gezeigt ist, hat der Steg eine gekrümmte Auflagefläche zur besseren seitlichen Fixierung der Drahtsonde. Außerdem sollte die Reibung zwischen Sonde 5 und Auflagefläche 6 nicht zu groß sein. Durch eine weitere Durchführung 7 wird die Sonde durch einen Griff 8 der Antriebsvorrichtung an der anderen Seite wieder herausgeführt. Das Gehäuse weist seitlich Schlitze 9 auf, die sich im wesentlichen senkrecht zu dem Steg 6 erstrecken. In die Schlitze 9 sind seitliche Achsbolzen 4 eines Rades 2 eingepaßt, so daß sich das Rad 2 entlang der Erstreckungsrichtung der Nuten 9 bewegen kann.

Das Rad 2 weist, wie am besten in den Figuren 2 und 3 gezeigt ist, eine zirkuläre umfangsseitige, konische, sich zur Mitte hin verjüngende Nut 3 auf. Der Winkel der Nutwände gegenüber der Senkrechten auf die Drehachse beträgt zwischen 1° und 6°, vorzugsweise zwischen 3° und 4°. Die Nutweite und die Position der Schlitze 9 gegenüber der Auflagefläche 6 sind so gewählt, daß die Sonde 5 von den Nutwänden gegen den Steg 6 angedrückt wird und so in der Nut 3 reibschlüssig gehalten wird. Aufgrund des gewählten Kleinen Nutwinkels ist die Reibungskraft zwischen Rad 2 und Sonde 5 so groß, daß schon die Gewichtskraft des Rades 2 alleine ein Mitführen der Sonde 5 beim Drehen des Rades 2 ermöglicht. Schon bei leichtem Andrücken des Rades können Zug- und Schubkräfte auf die Sonde 5 ausgeübt werden, die durch manuelles Schieben oder Ziehen der Sonde nicht möglich sind. Zur Vereinfachung der Betätigung kann die Umfangsfläche des Rades 2 (nicht gezeigt) Quernuten aufweisen.

Eine noch verbesserte Kraftübertragung vom Rad 2 auf die Sonde 5 kann erreicht werden, wenn die Schlitze 9, in denen sich die Drehachse des Rades 2 bewegt, nicht exakt senkrecht sondern in einem kleinen Winkel zur Senkrechten gegenüber dem Steg 6 angeordnet sind.

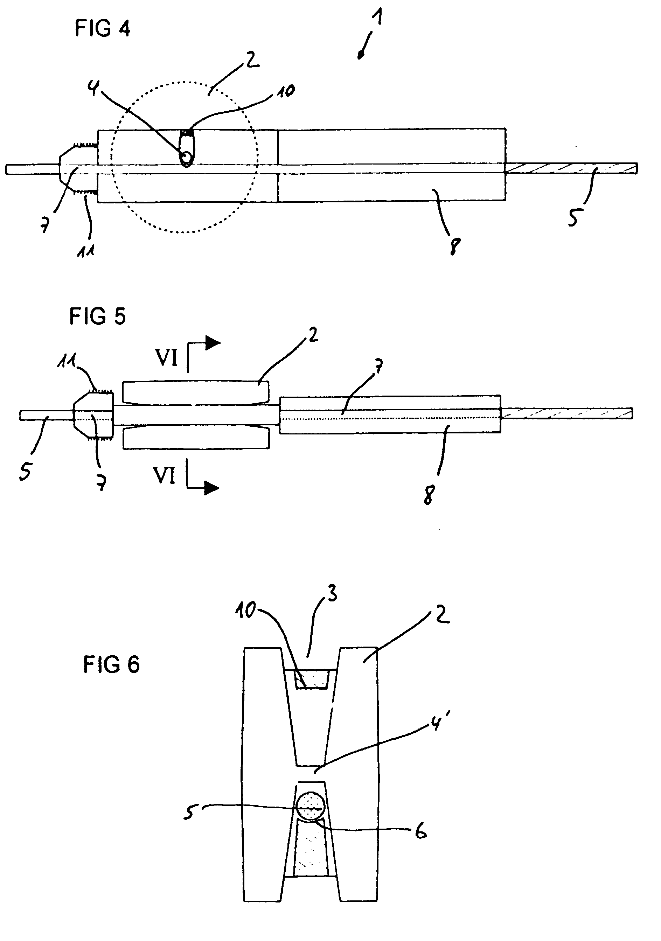

Die Figuren 4 bis 6 zeigen ein zweites Ausführungsbeispiel der erfindungsgemäßen Antriebsvorrichtung. Dabei ist das Rad nicht mittels äußerer Achsbolzen, sondern über eine Mittelachse 4' gegenüber dem Gehäuse bewegbar gelagert. Dies ist am besten in Figur 6 zu erkennen. Das Rad 2 ist zwischen Steg 6 und einem oberen Anschlag 10 bewegbar. Das Rad 2 ist so von allen Seiten zugänglich, was die Handhabbarkeit der erfindungsgemäßen Antriebsvorrichtung weiter verbessert. In Figur 4 ist aus Gründen der Darstellung das Antriebsrad 2 nur mit gestrichelter Linie angedeutet. Die Funktionsweise des in Figuren 4 bis 6 gezeigten Ausführungsbeispieles entspricht dem anhand der Figuren 1 bis 3 erläuterten ersten Ausführungsbeispiel.

Die in den Figuren 1 und 2 bzw. 4 und 5 gezeigten Antriebsvorrichtungen 1 weisen eine schematisch dargestellte Fixierungsvorrichtung 11 für eine Hohlsonde auf. So kann eine Hohlsonde fest am Gehäuse der Antriebsvorrichtung 1 fixiert werden, während das Zugseil einer Polypektomie-Schlinge in die Durchführungen 7 eingeschoben wird. Über das Rad 2 läßt sich so die Polypektomie-Schlinge mit einer Hand über einen sehr großen Hubweg betätigen.

Wird andererseits ein feststehender Führungsdraht in die Antriebsvorrichtung eingeführt, so kann durch Drehen des Rades 2 eine Relativbewegung zwischen dem Führungsdraht und einer an der Fixierungsvorrichtung 11 fixierten Hohlsonde erzeugt werden, wodurch die Hohlsonde gegenüber dem feststehenden Führungsdraht bewegt wird. So kann beispielsweise ein Drainagerohr mittels eines als Hohlsonde ausgebildeten Schiebeschlauches (Pusher) entlang des Führungsdrahtes durch das Endoskop in den Gallengang vorgeschoben werden.

Die erfindungsgemäße Antriebsvorrichtung ermöglicht so, daß die Sonde mit geringem Kraftaufwand definiert in beide Richtungen bewegt werden kann oder die Antriebsvorrichtung zum Vorschub von Instrumenten auf einem feststehenden Draht (Führungsdraht) verwendet werden kann.

## Patentansprüche

1. Antriebsvorrichtung für eine elastische Sonde, insbesondere eine medizinische Sonde, mit einem Handgriff und einem drehbaren Betätigungselement,
dadurch gekennzeichnet,
daß eine Auflagefläche (6) für die Sonde (5) vorgesehen ist und das Betätigungselement als Rad (2) mit einer zirkulären, konischen, sich zur Radmitte hin verjüngenden Nut (3) ausgebildet ist, wobei das Rad (2) so gegenüber der Auflagefläche (6) der Sonde (5) bewegbar ist, daß die Sonde (5) mit den Seitenwänden der konischen Nut (3) des Rades (2) gegen die Auflagefläche (6) andrückbar ist.

2. Antriebsvorrichtung nach Anspruch 1, **dadurch gekennzeichnet,** daß der Handgriff ein Gehäuse mit seitlichen Schlitzen (9) zur Aufnahme von seitlichen Achsenbolzen (4) des Rades (2) ist.

3. Antriebsvorrichtung nach Anspruch 2, **dadurch gekennzeichnet,** daß die Schlitze (9) im wesentlichen senkrecht zur oder mit einem Kleinen Winkel zur Senkrechten auf die Auflagefläche (6) der Sonde (5) angeordnet sind.

4. Antriebsvorrichtung nach Anspruch 1, **dadurch gekennzeichnet,** daß das Rad (2) mit einem mittleren Achsabschnitt (4') bewegbar an einem Gehäuse angebracht ist.

5. Antriebsvorrichtung nach einem der Ansprüche 2 bis 4, **dadurch gekennzeichnet,** daß das Gehäuse Durchführungen (7) für die elastische Sonde (5) aufweist.

6. Antriebsvorrichtung nach einem der Ansprüche 1 bis 5, **dadurch gekennzeichnet,** daß die Auflagefläche (6) für die Sonde (5) durch einen Steg gebildet wird, dessen Breite kleiner ist als der Außendurchmesser der elastischen Sonde (5).

7. Antriebsvorrichtung nach Anspruch 6, **dadurch gekennzeichnet,** daß der Auflagesteg (6) eine gekrümmte Auflagefläche hat.

8. Antriebsvorrichtung nach einem der Ansprüche 2 bis 7, **dadurch gekennzeichnet,** daß das Rad (2) austauschbar am Gehäuse befestigt ist.

9. Antriebsvorrichtung nach einem der Ansprüche 1 bis 8, **dadurch gekennzeichnet,** daß die Seitenwände der konischen Nut (3) einen Winkel von 1° bis 6° zur Senkrechten auf die Drehachse des Rades (2) aufweisen.

10. Antriebsvorrichtung nach Anspruch 9, **dadurch gekennzeichnet,** daß die Seitenwandwinkel zwischen 3° und 4° betragen.

11. Antriebsvorrichtung nach einem der Ansprüche 1 bis 10, **dadurch gekennzeichnet,** daß das Rad (2) eine gerändelte Umfangsfläche hat.

12. Antriebsvorrichtung nach einem der Ansprüche 1 bis 11, **dadurch gekennzeichnet,** daß am Gehäuse eine Fixierungsvorrichtung (11) für eine Hohlsonde ausgebildet ist.
